# EUROPÄISCHE PATENTSCHRIFT

(11) **EP 0 301 316 B1**
(45) Veröffentlichungstag und Bekanntmachung des Hinweises auf die Patenterteilung: **23.06.1993**
(21) Anmeldenummer: 88111231.2
(22) Anmeldetag: 13.07.1988
(51) Int. Cl.: C12N 15/00, A01H 1/00, A01G 7/00

(54) **Verfahren zum Gentransfer in Pflanzen**
Method of gene transfer in plants
Méthode de transfert de gènes dans des plantes

(30) Priorität: 21.07.1987 DE 3724154
(43) Veröffentlichungstag der Anmeldung: 01.02.1989
(73) Patentinhaber: AGROLINZ AGRARCHEMIKALIEN GESELLSCHAFT M.B.H., A-4021 Linz (AT)
(72) Erfinder: Heberle-Bors, Erwin, Dr., A-1180 Wien (AT); Benito Moreno, Rosa Maria, A-1200 Wien (AT); Alwen, Anna, Dipl.-Ing., A-1190 Wien (AT)
(74) Vertreter: Kunz, Ekkehard, Dr.

(56) Entgegenhaltungen:
- EP-A- 0 164 575
- EP-A- 0 257 472
- EP-A- 0 267 159
- EP-A- 0 270 356
- EP-A- 0 275 069
- EP-A- 0 286 429
- WO-A-85/01856
- APPLIED AND FUNDAMENTAL ASPECTS OF PLANT CELL, TISSUE AND ORGAN CULTURE, 1977, Seiten 506-535,563-577; Kapital 3, D. HESS: "Cell modfication by DNA uptake"
- CHEMICAL ABSTRACTS, Band 105, 1986, Seite 429, Zusammenfassung Nr. 187730q, Columbus, Ohio, US; M. KYO et al.: "Control of the developmental pathway of tobacco pollen in vitro"
- BIOLOGICAL ABSTRACTS/RMM, Zusammenfassung Nr. 33117342, Philadelphia, US; R.M.B. MORENO: "Gametophytes and sporophytes from tobacco microspores"
- NATURE, Band 325, Nr. 6101, 15.-21. Januar 1987, Seiten 274-276, Neptune, New York, US; A. DE LA PENA et al: "Transgenic rye plants obtained by injecting DNA into young floral tillers"
- PROC. NATL. ACAD. SCI, Band 83, Februar 1986, Seiten 715-719, US; Y. OHTA: "High-efficiency genetic transformation of maize by a mixture of pollen and exogenous DNA"
- BIOTECHNOLOGY AND ECOLOGY OF POLLEN; INTERNATIONAL CONFERENCE, Amherst, Mass., 9.-11. Juli 1985, Seiten 71-76, Springer-Verlag, New York, US; J.C. SANFORD et al.: "Attempted pollen-mediated transformation using Ti-plasmids"
- THE EMBO JOURNAL, Band 3, Nr. 12, 1984, Seiten 2717-2722, IRL Press Ltd, Oxford, GB; J. Paszkowski et al.: "Direct gene transfer to plants"
- PLANTA, Band 170, Nr. 1, Januar 1987, Seiten 141-143, Springer-Verlag, New York, US; D.R. PAREDDY et al.: "Fertilization and seed production with pollen from in-vitro-cultured maize tassels"
- GENETIC MANIPULATION IN PLANT BREEDING, PROC. INT. SYMP., 8.-13. September 1985, Seiten 803-811, Walter de Gruyter & Co., Berlin, DE (ed. 1986); D. HESS: "The pollen system of gene transfer"

## Beschreibung

Die Erfindung betrifft ein Verfahren zum Gentransfer in durch Bestäubung vermehrbaren Pflanzen mittels isolierter, unreifer und in vitro kultivierter Pollenkörner, die zur Bestäubung von Empfängerpflanzen und damit zur gewinnung von transformiertem Samen verwendet werden.

Es existieren schon verschiedene Techniken des Gentranfers in Pflanzen. Jede hat ihre Vorteile und Nachteile (Goodman et al, Science 236: 48-53, 1987). Agrobacterium tumefaciens ist derzeit der gebräuchlichste Vektor. Er ist jedoch begrenzt auf einen bestimmten Wirtsbereich. Immer noch hängt die Verwendung von A. tumefaciens als Vektor von der Regeneration in vitro kultivierter somatischer Zellen ab, um transgenische Pflanzen zu produzieren, die in der Lage sind, transgenische Nachkommen zu bilden. Direkter Gentransfer durch Elektroporation, Liposomen, Mikroinjektion und andere physikalisch-chemische Methoden hängt weithin von der Verwendung von Protoplasten als Zielzellen ab. Regeneration aus Protoplasten ist jedoch schwierig und sogar bei vielen Arten noch unmöglich.

Als eine Alternative zu diesen Gentransfermethoden wurde eine andere Zielzelle vorgeschlagen, das Pollenkorn. Nach Hess D., Plenum Press, New York, 519-537 (1975), ist es dem reifen Pollen möglich, Fremd-DNA aufzunehmen und als "Supervektor" diese Fremd-DNA In die Eizelle mit Hilfe natürlicher Bestäubung und Befruchtung einzubringen. Auf ähnliche Weise soll röntgenbestrahlter Pollen in der Lage sein, Bruchstücke des bestrahlten Genoms in die Eizelle zu transportieren (Pandey, Nature 256: 310-313, 1975). DeWet (WO 85/01856) berichtet, daß Maispollen, der mit exogener DNA behandelt wurde, diese DNA bei der Keimung aufnimmt und nach Bestäubung in die Eizelle transportiert.

Trotz der potentiellen grossen Bedeutung des Gentransfers in und durch Pollen gelang es bislang noch in keinem Fall, die Ergebnisse von Hess, Pandey und DeWet in anderen Labors zu reproduzieren. So konnte zum Beispiel Engvild (Theor Appl. Genet 69: 457-461, 1985) die Versuche von Pandey nicht reproduzieren. Bei den Versuchen von Hess (1975) und von DeWet ist die Aufnahme exogener DNA in das Pollenkorn und der genetische und molekulare Nachweis der transferierten DNA der kritische Punkt der Beweisführung. Phänotypischer und physiologischer Nach-weis reichen nicht aus (Hess in: Genetic manipulation in plant breeding, de Gruyter, Berlin, New York 803-811, 1986). Auch die Versuche von Sanford et al (Biotechnology and ecology of pollen (Mulcahy D, ed.), Springer, Heidelberg, New York, 71-75, 1985) zeigten, daß Kokultivierung von reifen Nicotiana langsdorfii-Pollen mit Agrobakterien zu keinem Gentransfer in Pollen führte. In umfangreichen Versuchen von Negrutiu, Heberle-Bors und Potrykus (ebendort 65-70, 1986) gelang es nicht, das Neomycinphosphotransferase-Gen, das Resistenz gegen Kanamycin verleiht, (Shillito et al, Biotechnology 3: 1099-1103, 1985) in reife Pollen zu übertragen. Mit Methoden, die dem neuesten Stand der Technik entsprechen, war es also nicht möglich, die Behauptungen von Hess und DeWet zu bestätigen. Daß die Ergebnisse von Hess und DeWet nicht nachvollziehbar sind, kann dadurch erklärt werden, daß reife Pollenkörner sofort mit der Bildung eines Pollenschlauches beginnen, sobald sie in ein für den Gentransfer notwendiges wäßriges Milieu gelangen. Offenbar sind sie dann nicht mehr befruchtungsfähig, bzw. es ist die Zeit, die für den Gentransfer zur Verfügung steht, zu kurz.
Pareddy et al beschreiben in Planta 170: 141-143 (1987), die Kultivierung und Reifung unreifer Maisfahnen ("tassels", männl. Blütenstände) in vitro. Mit dem isolierten reifen Pollen wurde bestäubt, und aus den bestäubten Pflanzen wurden Samen gewonnen. Ein mittels genetischer Markierung geführter Beweis für erfolgreiche Befruchtung konnte nicht erbracht werden.

Unerwarteterweise wurde gefunden, daß die Kultivierung unreifer Pollenkörner ohne das diese umhüllende natürliche Nährgewebe möglich ist, daß weiters in diese isolierten unreifen Pollenkörner während verschiedener Stadien der Reifung Fremdgene eingebracht werden können, und daß mit den ausgereiften Pollenkörnern Pflanzen bestäubt, befruchtet, zur normalen Samenbildung, zur Keimung und zur Vermehrung gebracht werden können.

Gegenstand der vorliegenden Erfindung ist demnach ein Verfahren zum Gentransfer in durch Bestäubung vermehrbaren Pflanzen, das dadurch gekennzeichnet ist, daß man
a) aus Staubgefäßen unreife Pollenkörner in einer Nährlösung, die alle für die Kultivierung und Aufrechterhaltung der Reifungsfähigkeit der Pollenkörner essentiellen Nähr- und Wuchsstoffe enthält, entnimmt und das umgebende Gewebe entfernt,
b) die isolierten unreifen Pollenkörner in einer Nährlösung, die für die Kultivierung und Aufrechterhaltung der Reifungsfähigkeit essentiellen Nähr-und Wuchsstoffe enthält, kultiviert
c) in die Pollenkörner während der in vitro-Kultivierung und Reifung fremdes Genmaterial überträgt
d) die transformierten Pollenkörner in vitro zur vollständigen Reifung bringt
e) mit den transformierten Pollenkörnern Empfängerpflanzen bestäubt und aus diesen Samen gewinnt.

Durch den Gentransfer in isolierte, unreife Pollenkörner, die in vitro-Reifung und die Verwendung von Pollen als universellen Supervektor wird eine völlig neue Strategie des Gentransfers in Pflanzen eröffnet. Im Vergleich zu anderen Methoden ist sie wesentlich vereinfacht, da die Zellkulturphase stark verkürzt wird und die Regeneration mit der unangenehmen Begleiterscheinung der somaklonalen Variation wegfällt. Mit dieser neuen Methode können auch Pflanzen transformiert werden, die einem erfolgreichen Gentransfer bisher nicht zugänglich waren: So sind z. B. viele Arten von Getreiden, Leguminosen und Bäumen nicht aus Einzelzellen oder Protoplasten regenerierbar.

Der potentielle Nutzen aus der Gentransfertechnik ist von grossem wirtschaftlichen, ökologischen und sozialen Wert. Es wird angestrebt, Pflanzen so genetisch zu verändern, daß der Ertrag erhöht wird, daß die Pflanzen resistent gegen Krankheiten und Schädlinge werden, daß sie gegen Kälte, Hitze, Trockenheit, Versalzung, Nährstoffmangel tolerant werden, daß sie eine größere Ernährungsqualität erlangen, daß sie neuartige lndustrierohstoffe produzieren, oder daß sie ihren eigenen Stickstoff fixieren oder auf andere Weise von Düngern unabhängig werden.

Wesentlich für die Erfindung ist, daß Pollenkörner ohne das sie umhüllende Gewebe, also isoliert, kultiviert werden, sodaß das zu übertragende Genmaterial in an einen Vektor gekoppelter oder in nackter Form direkten Kontakt zum Pollenkorn hat. Wird nämlich der komplette männliche Blütenstand kultiviert, ist es wegen der Behinderung durch das umfangreiche umhüllende Gewebe nicht möglich, mit Hilfe gängiger Transfermethoden (A. tumefaciens, Elektroporation, Mikroinjektion und dergleichen) Gene so in die Pollenkörner zu transferieren.

Ein weiteres wesentliches Erfindungsmerkmal ist die Verwendung unreifer Pollenkörner. Dadurch steht die gesamte Zeit der in vitro-Reifung zum Gentransfer zur Verfügung. Die Übertragung in das unreife Pollkorn kann in verschiedenen Reifestadien, abhängig von der Pflanzenart erfolgen. Insbesondere beim Gentransfer mit Vektoren kommt es darauf an, daß das Genmaterial möglichst wenig Zellwände überwinden muß, um in das Genom der Spermakerne zu gelangen und bei Befruchtung Teil des Zygotengenoms zu werden. Die Übertragung erfolgt bevorzugt bei einkernigen Mikrosporen, kann aber auch während der ersten Pollenmitose, im frühen zweikernigen Stadium, solange die generative Zelle noch an der Pollenwand haftet, oder auch zusätzlich bei jenen Pflanzen, die im Reifestadium dreikernige Pollen aufweisen (Getreide), im Stadium kurz vor oder während der zweiten Pollenmitose erfolgen.

Im einzelnen wird das Verfahren wie folgt durchgeführt, wobei als Modellsystem Tabak (Nicotiana tabacum) verwendet wurde. Das System ist auf alle durch Bestäubung vermehrbaren Pflanzen anwendbar, insbesondere auf ein- und zweikeimblättrige Kulturpflanzen wie Weizen, Mais, Reis, Leguminosen, Ölsaaten, Gemüsepflanzen, Obst- und Forstpflanzen.

In einem Vorversuch wird das Entwicklungsstadium der Pollen der gewünschten Pflanze in üblicher Weise durch Isolierung von einer Anthere, Herstellung eines Quetschpräparates und Beobachtung im Mikroskop nach Zusatz von z. B. Karminessigsäure bestimmt.

Haben die Pollen das gewünschte Stadium erreicht, werden die Pollenkörner unter aseptischen Bedingungen isoliert, indem man sie in einem Nährmedium aus den Antheren ausquetscht, durch ein Sieb passiert, wäscht, abzentrifugiert und resuspendiert.

Die Zelldichte soll für Pollenkörner im jüngeren Entwicklungsstadium höher sein, als für Pollenkörner im älteren Entwicklungsstadium. Sie beträgt beim Tabak von 2kernigen Pollenkörnern etwa 10⁵/ml, für einkernige Mikrosporen ist sie etwa doppelt so hoch.

Das verwendete Nährmedium enthält alle für die Kultivierung und Aufrechterhaltung der Reifungsfähigkeit der Pollenkörner essentiellen Nähr- und Wuchsstoffe. Je nach Pflanze können sich dabei unterschiedliche Zusammensetzungen ergeben, sodaß es die Funktion des Nährgewebes (Tapetum) erfüllt. Als Hauptbestandteile enthält das Nährmedium dabei Zucker, Nährstoffe, Mineralsalze und Vitamine, wobei der pH-Wert etwa 6,5 bis 7,5 beträgt.

Im Falle von einkernigen Mikrosporen erfolgt die Kultivierung bis zum Zweikernstadium in einem an Zucker z. B. Saccharose und weiteren Nährstoffen wesentlich angereicherten Medium. Beispielweise können die zusätzlichen Nährstoffe in Form von Kokosnußwasser zugeführt werden. Haben die Pollenkörner das zweikernige Stadium erreicht, können sie in ein weniger nährstoffreiches Medium überführt werden.

Nach beendeter in vitro-Reifung werden die Pollenkörner für die Bestäubung gewonnen. Dazu werden sie abzentrifugiert, gewaschen und in einem wäßrigen Medium oder getrocknet auf Blüten, aus denen vorher die Antheren entfernt wurden, zur Bestäubung aufgebracht. Aus den mit den in vitro gereiften Pollenkörnern bestäubten Pflanzen können auf allgemein übliche Art Samen gewonnen werden, welche keimungsfähig sind.

Zum Gentransfer während der in vitro-Kultivierung kann das zu übertragende fremde Genmaterial in Verbindung mit einem üblichen Vektor, wie z. B. A. tumefaciens oder als nackte DNA durch Direkttransfer mittels Elektroporation, Mikroinjektion oder andere physikalisch-chemische Methoden in die Pollenkörner eingebracht werden. Der Ausdruck "fremdes Genmaterial" bedeutet jedes außerhalb des zu transformierenden Pollenkorns entstandene Genmaterial. Nach erfolgter Reifung werden die Pollenkörner in der oben beschriebenen Weise zur Bestäubung gewonnen.

Als Beweis für die erfolgreiche in vitro-Reifung wurden Pollenkörner einer Tabakpflanze mit 2 Markergenen (KAN^{R}, NOS) in vitro gereift und mit diesen Pollenkörnern normale Wildtyppflanzen bestäubt. Die gewonnenen Samen wurden sterilisiert und in einem kanamycinhältigen Keimungsmedium zur Keimung gebracht. Die Mendel'sche Segregation der Markergene wurde durch Auszählung der Keimlinge im selektiven Medium nachgewiesen. Keimlinge, die auf kanamycinfreiem Medium gekeimt wurden, zeigten nach Nopalin-Nachweis in der Hochspannungspapierelektrophorese ebenfalls eine Mendel'sche Segregation des NOS-Gens. Dies ist der Beweis, daß es die in vitro-gereiften Pollenkörner waren, die die Befruchtung durchführten und nicht irgendwelche Pollenkontaminationen von anderen Pflanzen.

Als Beweis für die Expression des durch Tranformation während der in vitro-Kultivierung eingebrachten Fremdgens wurde in einem Enzymtest die Chloramphenicolacetyltransferase-Aktivität (CAT-Aktivität) in einem Homogenat aus den transformierten Pollenkörner nachgewiesen.

Zusätzlich zum CAT-Gen wurde auch ein cytochemisch nachweisbares Gen, das beta-Glucuronidase-Gen (GUS-Gen) verwendet. Dabei wurden die Agrobakterien nach der Kokultivierung mit den Pollen durch tägliches Waschen mit dem Antibiotikum Claforan entfernt oder abgetötet. Im cytochemischen Test (Blaufärbung der Pollen) konnten über 50 % blaue, in vitro gereifte Pollen nachgewiesen werden. Nicht infizierte Pollen, oder Pollen, die mit Agrobakterien ohne, bzw. mit nicht funktionellem GUS-Gen infiziert wurden, zeigten nach Substratzugabe keine Blaufärbung. Ein fluorimetrischer Test zeigte starke GUS-Aktivität in GUS-transformierten Pollen, wie auch in Pollen von GUS-transformierten Pflanzen, die als positive Kontrolle dienten. Pollen, die nicht oder mit GUS-Gen-freien Agrobakterien infiziert wurden, zeigten keine GUS-Aktivität. Auch Infektion mit Agrobakterien ohne Infektionsgene, aber mit komplettem GUS-Gen in der T-DNA führte zu keiner GUS-Aktivität in den Pollen (cytochemisch wie fluorimetrisch).

Daraus ist zu schließen, daß der Gentransfer in die Pollen durch Agrobakterium tumefaciens erfolgte. Ein weiterer Hinweis für erfolgreichen Gentransfer in die Pollen ist die erkennbare Anheftung von Agrobakterien an die Pollenoberfläche mit Bildung von Zellulosefibrillen (elektronenmikroskopische Aufnahmen).

### Beispiel 1: Bestimmung des Pollenentwicklungsstadiums

Tabakblüten wurden in verschiedenen Längen geerntet, die Antheren aseptisch isoliert, eine der fünf Antheren zusammen mit einem Tropfen Karminessigsäure (4 % Karmin in 45 % Essigsäure) auf einen Objektträger gebracht und ein Quetschpräparat hergestellt. Das Entwicklungsstadium der Pollenkörner wurde nach einer halben Stunde unter dem Mikroskop bestimmt.

### Beispiel 2: Isolierung der Pollenkörner

Die Tabakpollenkörner wurden isoliert, indem Antheren unter aseptischen Bedingungen in AMGLU-Medium (1) vorsichtig mit Glasstab und Mikroskopiernapf ausgequetscht, und die resultierende Pollensuspension durch ein 75-µm-Sieb passiert und zweimal in AMGLU-Medium gewaschen wurde. Zuletzt wurde die Pollensuspension bei 6500 U/min in einer Eppendorf Zentrifuge abzentrifugiert.

### Beispiel 3: Pollenkultur zur Reifung früh zweikerniger Pollenkörner

Früh zweikernige Tabakpollenkörner wurden isoliert und die Zelldichte in AMGLU-Medium auf 10⁵/ml eigestellt. Ein ml der Pollensuspension wurde in 35-mm-Petrischalen bei 25 °C in Dunkelheit kultiviert. Nach 2 bis 5 Tagen, abhängig vom genauen Entwicklungsstadium der Pollenkörner, waren die Pollenkörner reif und konnten geerntet werden.

### Beispiel 4: Pollenkultur zur Reifung einkerniger Mikrosporen

Einkernige Tabakmikrosporen wurden in MR24-Medium (2) bei einer Zelldichte von 2.10⁵/ml kultiviert. Sobald die Pollenkörner das zweikernige Stadium erreicht hatten (nach 2 bis 5 Tagen abhängig vom genauen Alter), wurden sie abzentrifugiert und in M1S-Medium (3) weiterkultiviert, bis die Reifung vollendet war.

Sehr gute Ergebnisse wurden auch bei Kultivierung in MR26-Medium (2′) erzielt. Sobald die Pollenkörner das zweikernige Stadium erreicht hatten (nach 3 Tagen), wurde dasselbe Volumen M2S-Medium dazugegeben. Nach einem weiteren Tag wurden die Pollenkörner abzentrifugiert und in M2S-Medium (3′) bei einer Zelldichte von 10⁵/ml weiterkultiviert, bis die Reifung vollendet war (ein Tag).

### Beispiel 5: Bestäubung und Nachweis der Befruchtung

Die Tabakpollenkörner wurden abzentrifugiert, in BK-Medium (Brewbaker und Kwack 1963) gewaschen und die Zelldichte auf 1.25.10⁶/ml eingestellt. Aus Blüten, die sich gerade öffneten (rote Blütenspitze) wurden die noch geschlossenen Antheren entfernt. Sobald die Blüte sich geöffnet hatte, wurde ein 4-µl-Tropfen der Pollensuspension mit Hilfe einer 20-µl-Pipette so auf die Narbe der Blüte aufgebracht, dass die Narbe komplett mit Pollensuspension bedeckt war. Diese Operationen wurden bei Bedingungen ohne Luftbewegung und von anderen Pflanzen derselben Art entfernt, durchgeführt. Sobald der Tropfen auf der Narbe angetrocknet war, wurden Narbe und Griffel mit einem 4 cm langen Strohhalm bedeckt, um Fremdbefruchtung zu verhindern. Die Pflanzen wurden dann ins Glashaus zurückgebracht.

### Beispiel 6: Samenernte, Samenkeimung und genetischer Test

Als Beweis, daß die in vitro maturierten Pollenkörner die Bestäubung und Befruchtung durchgeführt hatten, wurden Pollenkörner einer transgenischen Pflanze in vitro maturiert und mit diesen Pollenkörnern normale Wildtyppflanzen bestäubt. Als Markergen enthielt die transgenische Pflanze das Neomycin-Phospho-Transferase-Gen (Resistenz gegen Kanamycin) und das Nopalinsynthase-Gen. Selbstbefruchtungen und die reziproke Kreuzung mit in vitro gereiften Wildtyppollen wurden ebenfalls durchgeführt.

Die nach Beispiel 5 erhaltenen reifen Samenkapseln (braun und trocken) wurden geerntet und die Samen isoliert, indem die Spitze der Kapsel abgeschnitten und die Samenkörner direkt in ein Eppendorfhütchen eingefüllt wurden. Die Samen wurden in einer NaOCl-Lösung (3 % freies Chlor) 5 min lang oberflächensterilisiert, zweimal mit sterilem Wasser gewaschen und auf ein Samenkeimungsmedium mit Kanamycin (4) aufgelegt. Nach vier Wochen wurde die Zahl an Kan^{R} und Kan^{S} Keimlingen gezählt. Bei obigem Kreuzungsversuch ergaben die beiden reziproken Kreuzungen eine Segregation von Kan^{R} : Kan^{S} = 1 : 1. Keimlinge, die ohne Kanamycin aufwuchsen, zeigten nach einem Nopalintest durch Hochspannungspapierelektrophorese eine Segregation von Nos⁺ : Nos⁻ = 1 : 1. Die Selbstbefruchtung mit in vitro gereiften Wildtypen ergab wie erwartet eine 0 : 1 Segregation für beide Markergene. Die Selbstbefruchtung mit in vitro gereiften Pollen der transgenischen Pflanze ergab eine 3 : 1 Segregation.

### Beispiel 7: Transiente Expression des CAT-Gens

Agrobakterien (A.tumefaciens ohne Tumorgene, mit CAT-Gen gekoppelt an 35S-Promotor) wurden in Luria-broth einen Tag vorinkubiert. Pollenkörner im früh zweikernigen Stadium wurden isoliert und in AMGLU-Medium kultiviert. Die Bakteriensuspension wurde mit AMGLU-Medium auf eine OD₅₈₀ von 0.2 eingestellt. Nach einer weiteren Verdünnung mit AMGLU-Medium von 1 : 10 wurden 20 µl der Bakteriensuspension zu 1 ml der Pollensuspension gegeben. Nach 24 Stunden Kokultivierung wurde zur Abtötung der Agrobakterien 1 µl Claforan (1g/2 ml) pro ml zugegeben. Nach zwei weiteren Tagen wurden die Pollenkörner geerntet und ein Extrakt hergestellt. Dazu wurden pro ml Pollensuspension 1.5 ml Calcium-Waschlösung (5), pH-Wert 5.6 zugegeben, bei 4000 U/min 5 min zentrifugiert und mit einem Tris-Puffer (0.25 M, pH 7.8) gewaschen. Nach Zentrifugation wurde das Pollenpellet mit 200 µl Tris-Puffer versetzt, und durch Ultraschall (3 x 15 sec) auf Eis homogenisiert. Nach 10 min auf Eis wurde abzentrifugiert und der Überstand bei -20 °C aufbewahrt.

Der CAT-Test erfolgte wie von Sleigh (Anal. Biochem., 56: 251 - 256, 1986) beschrieben. 30 µl des Extrakts wurden mit 20 µl Chloramphenicol (8mM), 30 µl Tris-Puffer und 20 µl ¹⁴C-markiertes Acetyl-CoA (5 uCi/ml in 0.5 mM kaltem Acetyl-CoA) vermischt. Nach Inkubation bei 37 °C für 1 Stunde wurde das acetylierte Chloramphenicol durch Äthylacetat ausgeschüttelt (2 x 100 µl) und die Radioaktivität im Szintillationszähler gemessen.

| Radioaktivität (cpm) im CAT-Test von Extrakten aus Tabakpollen nach Kokultivierung mit A. tumefaciens | |
|---|---|
| | cpm |
| Pollen in AMGLU-Medium | 400 |
| Pollen mit Agrobakterien in AMGLU-Medium | 6 000 |
| Pollen mit Acetosyringon-aktivierten Agrobakterien in AMGLU-Medium | 6 000 |
| nur Agrobakterien in AMGLU-Medium | 500 |
| nur Acetosyringon-aktivierte Agrobakterien in AMGLU-Medium | 500 |

Das starke Radioaktivitätssignal zeigt, daß die Agrobakterien die Pollenkörner erfolgreich infiziert haben und die T-DNA zur Expression (Transkription) in den Zellkern der wachsenden Zelle gelangt sein muß.

Als weitere Kontrolle, um zu zeigen, daß die Agrobakterien selbst keine CAT-Aktivität hatten, zeigte eine Agrobakterienkultur in Luria-broth über einen kompletten Wachstumszyklus keine CAT-Aktivität.

### Beispiel 8: Expression des GUS-Gens in Tabakpollen

Agrobakterien des Stammes LBA 4404 (A. tumefaciens, disarmed, mit beta-Glucuronidase (GUS-) Gen, gekoppelt an 35S-Promotor und Terminator, (Matzke und Matzke in Plant Molecular Biology 7 : 357 - 365 (1986)), wurden in Luria-broth einen Tag vorinkubiert. Pollenkörner im spät einkernigen Stadium wurden isoliert und in MR26-Medium auf eine 0D₅₈₀ von 0.2 eingestellt. Nach einer weiteren Verdünnung mit MR26-Medium von 1 : 10 wurden 20 µl der Bakteriensuspension zu 1 ml der Pollensuspension gegeben. Nach 14 - 20 Stunden Kokultivierung wurde abzentrifugiert und die Pollen dreimal mit claforanhältigem (1 g/2 ml) MR26-Medium gewaschen und weiterkultiviert. Bis zur Reifung der Pollen wurde jeden Tag das claforanhältige Medium ausgewechselt. 40 µl des letzten Mediums (M2S mit Claforan) wurden zu einem Luria-broth gegeben. Es entwickelte sich kein Bakterienwachstum.
Die gereiften Pollen wurden abzentrifugiert und ein Teil in MR26-Medium aufgenommen. Nach Zugabe von X-Glu (5-Bromo-4-chloro-3-indolyl-Glucuronid) auf eine Endkonzentration von 1 mM und Inkubation bei 37 °C für 4 - 12 Stunden (Jefferson in Plant Molecular Biology Reporter, Vol. 5, Nr. 4, 387 - 405, (1987)) konnte die Bildung von Indigo (Produkt der beta-Glucuronidase aus X-Glu) anhand der Blaufärbung der Pollen im Lichtmikroskop nachgewiesen werden. Mehr als 50 % der lebenden, gereiften Pollen zeigte Blaufärbung.
Ein zweiter Teil der kokultivierten und in vitro gereiften Pollen wurde in GK-Medium (wie BK-Medium, aber doppelte Konzentration an Borsäure) gekeimt. In den Pollenschläuchen der gekeimten Pollen konnte ebenfalls GUS-Aktivität nachgewiesen werden.
In einem Kontrollversuch wurden Pollen mit einem Agrobakterienstamm von LBA4404 infiziert, der in seiner T-DNA das GUS-Gen ohne Promotor enthielt (von Drs. Matzke, Salzburg, zur Verfügung gestellt). Diese Pollen zeigten nach in vitro Reifung und X-Glu Zugabe keine Blaufärbung. Auch Agrobakterien, die kein GUS-Gen, sondern das KAN_{35S}-Gen enthielten, zeigten nach Kokultivierung mit Pollen und X-Glu-Zugabe keine Blaufärbung. Auch Pollen, die nicht mit Agrobakterien infiziert wurden, zeigten nach X-Glu-Zugabe keine Blaufärbung.

Pollen von einer GUS_{35S}-transformierten Pflanze (durch leaf-disk), die als positive Kontrolle dienten, zeigten Blaufärbung. Ein anderer Agrobakterienstamm, der zwar ein funktionelles GUS_{35S}-Gen in der T-DNA enthielt, aber in seiner Virulenzfunktion gestört war (binary vektor ohne Ti-Plasmid), zeigte keine GUS-Aktivität in den Pollen.

Von einem dritten Teil der Pollen wurde ein Extrakt hergestellt. Dazu wurden jeweils 4.10⁵ Pollen abzentrifugiert und in Extraktionspuffer (6) aufgenommen. Die Pollen wurden mit Glaskugeln und gleichzeitiger Ultraschallbehandlung aufgebrochen. Der fluorimetrische GUS-Test erfolgte wie von Jefferson beschrieben. 50 µl Extrakt wurden mit Extraktionspuffer auf 1 ml aufgefüllt und mit MUG (4-Methyl-Umbelliferyl-Glucuronid) auf eine Endkonzentration von 1 mM versetzt. Alle 10 oder 30 sec wurden 200 µl Aliquote entnommen und die Enzymreaktion mit 800 µl Na₂CO₃ (0,2 M) gestoppt. In einem Fluorimeter wurde die Extinktion bei 455 nm nach Anregung mit 365 nm gemessen und in Konzentrationen in µM/ml anhand von Standardlösungen an MUG umgerechnet.

| Fluorimetrisch gemessene Enzymaktivität in µM/ml nach 10, 20 und 30 Minuten der beta-Glucuronidase aus Tabakpollenextrakten nach Kokultivierung mit A. tumefaciens: | | | |
|---|---|---|---|
| | 10 min | 20 min | 30 min |
| Standard 1 | 1,0 | 1,0 | 1,0 |
| Standard 2 | 0,1 | 0,1 | 0,1 |
| Pollen ohne Agrobakterien | 0,016 | 0,020 | 0,019 |
| Pollen mit GUS_{35S} Agrobakterien kokultiviert | 0,057 | 0,078 | 0,109 |
| Pollen mit KAN_{35S} Agrobakterien kokultiviert | 0,023 | 0,019 | 0,021 |
| Pollen mit GUS_{35S} mit "binary vektor" ohne Ti-Plasmid | 0,022 | 0,023 | 0,018 |
| Pollen von transgenischer GUS⁺ Pflanze | 0,051 | 0,085 | 0,121 |

### Kulturmedien:

(1) AMGLU-Medium
   Miller Macrosalze
   MS Microsalze
   Saccharose (0.25 M)
   Glutamin (440 mg/l
   pH 7
(2) MR24-Medium
   MS Macrosalze
   MS Microsalze
   Saccharose (0.5 M)
   Glutamin (440 mg/l)
   Cocosnusswasser (2 Vol%)
   Lactalbumin Hydrolysat (200 mg/l)
   Inositol (100 mg/l)
   pH 7
(2′)MR26-Medium
   wie MR24-Medium, aber Lactalbumin-Hydrolysat (1 g/l)
(3) M1S-Medium
   Miller Macrosalze
   MS Microsalze
   FeEDTA (10⁻⁴M)
   Saccharose (0.25 M)
   pH 7
(3′) M2S-Medium
   Kyo und Harada Salze (in Planta 186: 427-432 (1986))
   Saccharose (0,25 M)
   pH 7
(4) Samenkeimungsmedium
   MS Macrosalze
   MS Microsalze
   FeEDTA (10⁻⁴ M)
   Saccharose (1 Gew%)
   Agar (0.8 Gew%)
   Kanamycin.SO₄ (50 mg/l)
   pH 5.5
(5) Calcium-Waschlösung
   CaCl₂.2H₂0 (0,16 M)
   MES-Puffer (0,5 Gew%)
   pH 5.6
(6) Extraktionspuffer
   NaPO₄ (50 mM, pH 7)
   2-Mercaptoäthanol (10 mM)
   Na₂EDTA (10 mM)
   Natrium-Lauryl-Sarcosin (0,1 %)
   Triton X-100 (0,1 %)

## Patentansprüche

1. Verfahren zum Gentransfer in durch Bestäubung vermehrbaren Pflanzen, dadurch gekennzeichnet, daß man
a) aus Staubgefäßen unreife Pollenkörner in einer Nährlösung, die alle für Kultivierung und Aufrechterhaltung der Reifungsfähigkeit essentiellen Nährund Wuchsstoffe enthält entnimmt und das umgebende Gewebe entfernt,
b) die Isolierten unreifen Pollenkörner in einer Nährlösung, die alle für die Kultivierung und Aufrechterhaltung der Reifungsfähigkeit essentiellen Nährund Wuchsstoffe enthält, kultiviert,
c) in die Pollenkörner während der in vitro-Kultivierung und Reifung fremdes Genmaterial überträgt
d) die transformierten Pollenkörner in vitro zur vollständigen Reifung bringt
e) mit den transformierten Pollenkörnern Empfängerpflanzen bestäubt und aus diesen Samen gewinnt.

2. Verfahren nach Anspruch 1, dadurch gekennzeichnet, daß die Übertragung fremden Genmaterials im Stadium einkerniger Mikrosporen erfolgt.

3. Verfahren nach Anspruch 1, dadurch gekennzeichnet, daß die Übertragung fremden Genmaterials während der ersten Pollenmitose erfolgt.

4. Verfahren nach Anspruch 1, dadurch gekennzeichnet, daß die Übertragung im früh zweikernigen Stadium, solange die generative Zelle noch an der Pollenwand haftet, erfolgt.

5. Verfahren nach Anspruch 1, dadurch gekennzeichnet, daß die Übertragung fremden Genmaterials kurz vor oder während der zweiten Pollenmitose erfolgt.

6. Verfahren nach Anspruch 2, dadurch gekennzeichnet, daß der Gentransfer im Stadium einkerniger Mikrosporen in einem an Zucker und weiteren Nährstoffen angereicherten Medium erfolgt.

7. Verfahren nach Anspruch 1, dadurch gekennzeichnet, daß die Übertragung fremden Genmaterials durch Kokultivierung mit Agrobacterium tumefaciens erfolgt.

## Claims

1. Method for transferring genes into plants which are capable of being propagated by pollination, characterised in that
a) immature pollen grains are extracted from stamens in a nutrient solution which contains all nutrients and growth substances essential for culturing and maintaining the maturability of the pollen grains, and the surrounding tissue is removed,
b) the isolated immature pollen grains are cultured in a nutrient solution which contains the nutrients and growth substances essential for culturing and maintaining the maturability,
c) foreign genetic material is transferred into the pollen grains during the in-vitro cultivation and maturation,
d) the transformed pollen grains are allowed to mature completely in vitro,
e) acceptor plants are pollinated with the transformed pollen grains, and seeds are obtained from these plants.

2. Process according to Claim 1, characterised in that the transfer of foreign genetic material takes place at the uninucleate microspore stage.

3. Process according to Claim 1, characterised in that the transfer of foreign genetic material takes place during the first pollen mitosis.

4. Process according to Claim 1, characterised in that the transfer takes place in the early-binucleate stage while the generative cell is still attached to the pollen wall.

5. Process according to Claim 1, characterised in that the transfer of foreign genetic material takes place just before, or during, the second pollen mitosis.

6. Process according to Claim 2, characterised in that the gene transfer takes place in the uninucleate microspore stage in a medium which is enriched with sugar and other nutrients.

7. Process according to Claim 1, characterised in that the transfer of foreign genetic material takes place by coculture with Agrobacterium tumefaciens.

## Revendications

1. Procédé de transfert de gène dans des plantes multipliables par fécondation, caractérisé en ce que :
(a) on prélève dans une étamine des grains de pollen immatures dans un milieu nutritif renfermant les matières nutritives et de croissance essentielles pour la culture et l'entretien des capacités de maturation des grains de pollen et on élimine le tissu environnant,
(b) on cultive les grains de pollen immatures isolés dans un milieu nutritif renfermant les matières nutritives et de croissance essentielles pour la culture et l'entretien des capacités de maturation,
(c) on transfère dans les grains de pollen du matériau génétique étranger durant la culture in vitro et la maturation,
(d) on réalise la maturation complète in vitro des grains de pollen transformés,
(e) on féconde les plantes recevantes avec les grains de pollen transformés et obtenus à partir de cette semence.

2. Procédé selon la revendication 1, caractérisé en ce qu'on réalise le transfert du matériau génétique étranger au stade des microspores à noyau unique.

3. Procédé selon la revendication 1, caractérisé en ce qu'on réalise le transfert du matériau génétique étranger durant la première mitose du pollen.

4. Procédé selon la revendication 1, caractérisé en ce qu'on réalise le transfert au stade précoce de deux noyaux aussi longtemps que les cellules génératives sont prisonnières de la paroi du pollen.

5. Procédé selon la revendication 1, caractérisé en ce qu'on transfère le matériau génétique étranger juste avant ou pendant la deuxième mitose du pollen.

6. Procédé selon la revendication 2, caractérisé en ce qu'on réalise le transfert génétique au stade des microspores à noyau unique dans un milieu enrichi en sucre et en d'autres substances nutritives.

7. Procédé selon la revendication 1, caractérisé en ce qu'on transfère le matériau génétique étranger par culture simultanée avec Agrobacterium tumefaciens.
